# EUROPÄISCHE PATENTSCHRIFT

(11) **EP 2 398 387 B1**
(45) Veröffentlichungstag und Bekanntmachung des Hinweises auf die Patenterteilung: **02.05.2018**
(21) Anmeldenummer: 10705110.4
(22) Anmeldetag: 17.02.2010
(51) Int. Cl.: A61B 5/151

(54) **ANALYSEHANDGERÄT**
HANDHELD ANALYSIS TOOL
APPAREIL MANUEL D'ANALYSE

(30) Priorität: 18.02.2009 EP 09002281
(43) Veröffentlichungstag der Anmeldung: 28.12.2011
(73) Patentinhaber: Roche Diabetes Care GmbH, 68305 Mannheim (DE); F. Hoffmann-La Roche AG, 4070 Basel (CH)
(72) Erfinder: RÖDEL, Wolfgang, 69123 Heidelberg (DE); MILTNER, Karl, 67227 Frankenthal (DE); BAETER, Thorsten, 67136 Fußgönheim (DE); FRISCH, Gerhard, 68535 Edingen-Neckarhausen (DE); LIEDTKE, Sebastian, 69117 Heidelberg (DE); HECK, Wolfgang, 67227 Frankenthal (DE)
(74) Vertreter: Twelmeier Mommer & Partner
(86) Internationale Anmeldenummer: PCT/EP2010/000994
(87) Internationale Veröffentlichungsnummer: WO 2010/094469

(56) Entgegenhaltungen:
- EP-A- 1 360 932
- EP-A- 1 891 898
- US-A1- 2006 178 600
- US-A1- 2007 255 301

## Beschreibung

Die Erfindung geht aus von einem Analysehandgerät mit den im Oberbegriff des Anspruchs 1 angegebenen Merkmalen.

Ein Analysehandgerät mit den im Oberbegriff des Anspruchs 1 angegebenen Merkmalen ist aus der US 2006/178600 A1 bekannt. Dieses Analysehandgerät ist in eine Armbanduhr integriert. In dem ersten Gerätezustand kann mit dem Gerät eine Körperflüssigkeitsprobe durch einen Lanzettenstich gewonnen und deren Glucosekonzentration ermittelt werden. In dem zweiten Gerätezustand dient das Gerät als Armbanduhr, indem mit der Anzeigeeinrichtung die aktuelle Uhrzeit angezeigt wird.

Analysehandgeräte werden insbesondere von Diabetikern benötigt, die mehrmals täglich ihren Blutzuckerspiegel überprüfen müssen und dazu eine Körperflüssigkeitsprobe, in der Regel Blut und/oder interstitielle Flüssigkeit, benötigen, die aus einer kleinen Stichwunde gewonnen wird. Mit einem in dem Analysehandgerät enthaltenen Sensor kann eine Analytkonzentration, beispielsweise die Glucosekonzentration, einer durch einen Lanzettenstich gewonnenen Körperflüssigkeitsprobe gemessen werden.

Ständiges Ziel bei der Entwicklung von Analysehandgeräten ist es, Benutzern eine möglichst einfache Handhabung und eine schnelle Durchführung einer Messung zu ermöglichen.

Aufgabe der Erfindung ist es deshalb, einen Weg aufzuzeigen, wie der Benutzerkomfort bei einem Analysehandgerät der eingangs genannten Art verbessert werden kann.

Diese Aufgabe wird durch ein Analysehandgerät mit den im Anspruch 1 angegebenen Merkmalen gelöst. Vorteilhafte Weiterbildungen der Erfindung sind Gegenstand von Unteransprüchen.

Ein erfindungsgemäßes Analysehandgerät kann mit einem ersten Betätigungselement in einen ersten aktiven Zustand versetzt werden, in dem eine mechanische Baugruppe mit einer Transporteinrichtung für analytische Verbrauchselemente, beispielsweise Testelemente und/oder Lanzetten, aktiviert und eine Messung vorbereitet wird. In dem ersten aktiven Zustand kann dann durch Betätigen eines der Betätigungselemente des Analysehandgeräts eine Messung gestartet werden. Ein erfindungsgemäßes Analysehandgerät hat zusätzlich einen zweiten aktiven Zustand, in dem die Anzeigeeinrichtung ohne Aktivieren der mechanischen Baugruppe eingeschaltet wird. In diesem zweiten aktiven Zustand werden insbesondere Ergebnisse früherer Messungen angezeigt, beispielsweise damit ein Benutzer seinen Behandlungsfortschritt überprüfen kann.

Beim Aktiveren der mechanischen Baugruppe wird mindestens ein Bauteil der Baugruppe bewegt, beispielsweise ein Testelement und/oder eine Lanzette aus einem Vorrat entnommen. Bei einfachen Analysehandgeräten muss die dafür erforderliche Kraft vom Benutzer aufgebracht werden, bei komplexeren Analysehandgeräten, deren mechanische Baugruppe einen Elektromotor enthält, wird ein elektrischer Energiespeicher, in der Regel eine Batterie, belastet. Vorteilhaft muss dieser Aufwand bei einem erfindungsgemäßen Gerät nur dann erfolgen, wenn ein Benutzer auch tatsächlich eine Messung durchführen möchte. Wenn ein Benutzer aber keine Messung durchführen möchte, sondern beispielsweise lediglich eine Serie von früheren Messungen betrachten oder eine Geräteeinstellung ändern, beispielsweise eine in das Gerät integrierte Uhr stellen möchte, kann der mit dem Vorbereiten einer Messung verbundene Aufwand vorteilhaft unterbleiben, insbesondere kann eine Bewegung mechanischer Komponenten unterbleiben.

Ein erfindungsgemäßes Analysehandgerät kann als ein reines Messgerät ausgebildet sein, das zusammen mit einem separaten Stechgerät ein Messsystem bildet. Bevorzugt ist in ein erfindungsgemäßes Analysehandgerät aber ein Stechgerät integriert. In einem solchen Fall enthält die mechanische Baugruppe zusätzlich auch einen Stechantrieb, um Lanzetten in eine Stechbewegung zu versetzen.

Die Verbrauchselemente können beispielsweise Testelemente mit Nachweisreagenzien zur Konzentrationsbestimmung, insbesondere zur fotometrischen oder elektrochemischen Konzentrationsbestimmung, oder Lanzetten sein. Die Verbrauchselemente können insbesondere auch Lanzetten mit integrierten Testelementen sein. Beispielweise können als Testelemente Lanzetten verwendet werden, die einen Testbereich aufweisen. Ein solcher Testbereich kann beispielsweise eine Küvette zur reagenzfreien Untersuchung oder ein Testfeld mit Nachweisreagenzien sein. Testfelder mit Nachweisreagenzien zur fotometrischen oder elektrochemischen Konzentrationsbestimmung werden bei handelsüblichen Teststreifen zur Glukosekonzentrationsbestimmung seit Jahrzehnten verwendet. Entsprechende Testfelder können auf eine Lanzette aufgeklebt sein. Eine solche Lanzette kann einen Kanal, beispielsweise in Form einer Rinne, aufweisen, der durch Kapillarkräfte einen Flüssigkeitstransport zu einem Testfeld bewirkt. Möglich ist es aber auch, in einem erfindungsgemäßen Analysehandgerät als Verbrauchselemente Lanzetten und separate Testelemente zu verwenden. Derartige Testelemente können eine Körperflüssigkeitsprobe von einer Lanzette aufnehmen, die zu diesem Zweck von der mechanischen Baugruppe des Analysehandgeräts zu dem Testelement geführt wird. Möglich ist es auch, dass separate Testelemente eine Körperflüssigkeitsprobe direkt von einer zuvor erzeugten Stichwunde aufnehmen.

Der Verbrauchselementevorrat für ein erfindungsgemäßes Analysehandgerät kann als ein in das Gerät einsetzbares Magazin ausgebildet sein. Ein derartiges Magazin kann Testelemente und/oder Lanzetten in separaten Magazinkammern enthalten. Möglich ist es auch, ein solches Magazin als eine Kassette mit einem aufgewickelten Trägerband auszubilden, das Testelemente und/oder Lanzetten oder Lanzetten mit integrierten Testelementen trägt. Bei einem Analysehandgerät das ein Messgerät und ein Stechgerät ist, wird vorzugsweise ein interner Vorrat von Lanzetten und Stechelementen verwendet. Möglich ist es aber beispielsweise auch, in dem Gerät nur einen Lanzettenvorrat vorzusehen und Testelemente nach einem Stich für eine Messung einzeln in das Gerät einzuführen.

Eine Messung kann beispielsweise vorbereitet werden, indem ein Testelement und/oder eine Lanzette aus einem Vorrat entnommen werden, indem ein mechanischer Energiespeicher eines Stechantriebs aufgeladen wird, oder indem ein Stechantrieb an eine Lanzette ankoppelt. Bei einem erfindungsgemäßen Gerät können zur Vorbereitung einer Messung alle diese Schritte oder nur ein Teil dieser Schritte durchgeführt werden. Beispielsweise ist es auch möglich, dass ein Testelement oder eine Lanzette nach Abschluss einer Messung aus einem Vorrat entnommen und in eine Gebrauchsposition gebracht werden, in der das Testelement bzw. die Lanzette bis zur nächsten Messung bleiben. Bevorzugt werden Testelemente und/oder Lanzetten aber erst bei der Vorbereitung einer Messung im ersten aktiven Zustand des Geräts aus einem Magazin entnommen, da diese in dem Magazin am besten vor schädlichen Umwelteinflüssen geschützt sind.

Eine vorteilhafte Weiterbildung der Erfindung sieht vor, dass die mechanische Baugruppe nach Aktivierung durch Betätigen des ersten Betätigungselements deaktiviert werden kann. Bevorzugt ist auch, dass sich das Gerät nach einer vorgegeben Zeitspanne selbsttätig deaktiviert, wenn bis dahin keine Messung durchgeführt wurde. Die vorgegebene Zeitspanne kann vom Hersteller des Geräts fest vorgegeben werden und beispielsweise zwischen einer halben und zehn Minuten, insbesondere zwischen zwei und fünf Minuten, betragen

Wenn eine Messung vorbereitet und die mechanische Baugruppe aktiviert wird, kann es nämlich vorkommen, dass ein Benutzer keine Messung durchführen möchte, beispielsweise weil die Aktivierung auf einem Versehen beruht oder der Benutzer es sich anders überlegt hat. Beim Aktivieren der mechanischen Baugruppe kann beispielsweise ein Testfeld oder eine Lanzette aus einer sterilen Verpackung entnommen werden. Nach einiger Zeit kann dann die Funktionsfähigkeit des Testfeldes oder die Sterilität der Lanzette nicht mehr gewährleistet werden. Durch Deaktivieren der mechanischen Baugruppe lässt sich sicherstellen, dass Testfelder und/oder Lanzetten nur zeitnah nach der Vorbereitung einer Messung verwendet werden. Kommt es nicht innerhalb der vorgegebenen Zeit zu einer Messung, wird das Testfeld und/oder die Lanzette spätestens bei einem erneuten Aktivieren der mechanischen Baugruppe weitertransportiert, beispielsweise in einen für benutzte Testfelder und/oder Lanzetten vorgesehenen Raum in dem Gerät.

Der Umgang mit einem unbenutzten Testfeld bereitet beim Deaktivieren der mechanischen Baugruppe keine besonderen Schwierigkeiten. Das Testfeld kann bis zum erneuten Aktivieren in seiner Position bleiben und wird einfach beim erneuten Aktivieren weiter bewegt. Bei einer Lanzette können wegen des Stechantrieb des Geräts aber zusätzliche Maßnahmen angebracht sein, insbesondere wenn das Aktivieren der mechanischen Baugruppe damit verbunden ist, dass ein mechanischer Energiespeicher des Stechantriebs aufgeladen wird, beispielsweise eine Antriebsfeder gespannt wird. Bevorzugt wird in einem solchen Fall der mechanische Energiespeicher beim Deaktivieren der mechanischen Baugruppe entladen. Bevorzugt geschieht dies, indem ein Stich ausgelöst wird.

Beispielsweise kann das erste Betätigungselement eine Abdeckung einer Geräteöffnung sein, an der eine Probenaufnahme erfolgt. Wird die Geräteöffnung freigelegt, aktiviert sich die mechanische Baugruppe. Wenn die Geräteöffnung wieder verschlossen wird, ohne dass zuvor ein Stich ausgelöst wurde, kann dadurch ein Stich ausgelöst werden. Bevorzugt weist das Betätigungselement, d .h. die Abdeckung, an der Innenseite eine Vertiefung für die Lanzette auf.

Möglich ist es aber auch, dass der Energiespeicher ohne Auslösen eines Stichs wieder entladen wird. Dies macht zwar eine etwas aufwendigere Mechanik des Stechantriebs erforderlich, ist aber in Bezug auf ein selbsttätiges Aktivieren vorteilhaft. Möglich ist es auch, dass sich das Gerät nach einer vorgegeben Zeitspanne selbsttätig deaktiviert, wenn bis dahin keine Messung durchgeführt wurde und dabei die Geräteöffnung verschießt, also das Betätigungselement automatisch bewegt wird. Nach Verschließen der Geräteöffnung kann ein Stich ohne Verletzungsgefahr für den Benutzer ausgelöst werden.

Weitere Einzelheiten und Vorteile der Erfindung werden anhand eines Ausführungsbeispiels unter Bezugnahme auf die beigefügten Zeichnungen erläutert. Es zeigen:
- Figur 1: eine schematische Darstellung eines tragbaren Blutzuckermessgeräts; und
- Figur 2: eine schematische Darstellung des in Figur 1 dargestellten Messgeräts mit aufgeschnittenem Gehäuse.

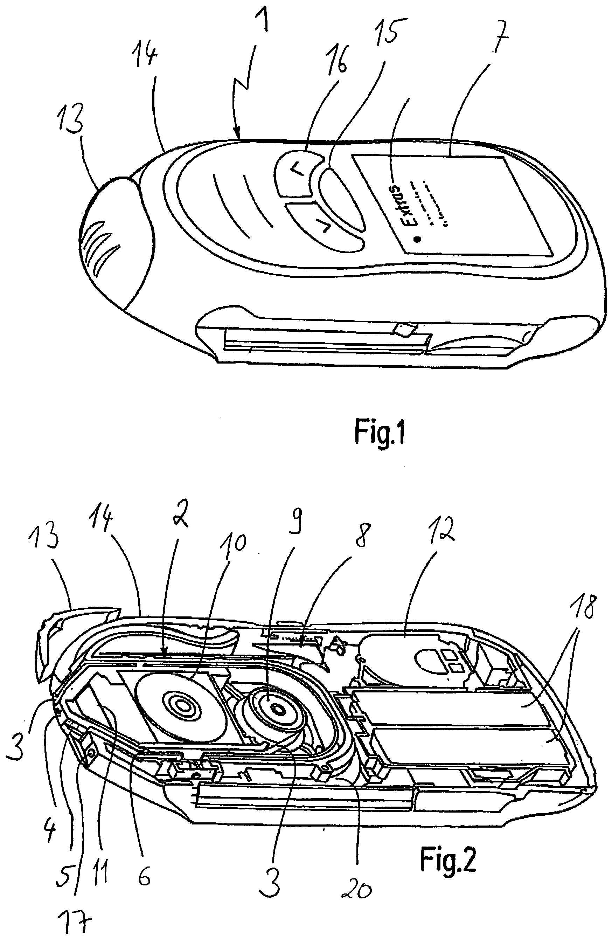

Das in den Figuren 1 und 2 dargestellte Analysehandgerät 1 ermöglicht einem Benutzer die Bestimmung der Glukosekonzentration einer Körperflüssigkeitsprobe in einem weitgehend automatischen Messablauf. In ein Aufnahmefach des Geräts 1 ist ein Magazin 2 mit einem Testelementvorrat einsetzbar. Hierfür ist ein bodenseitiger Gehäusedeckel 20 abnehmbar. Bei dem dargestellten Ausführungsbeispiel ist das Magazin 2 eine Bandkassette, die auf einem Trägerband 3 Lanzetten 4 und Testelemente 5 trägt. Anstelle einer solchen Kassette kann beispielsweise auch ein Magazin mit einzelnen Magazinkammern verwendet werden.

Eine durch einen Lanzettenstich gewonnene Körperflüssigkeitsprobe kann mit einem Sensor 6 untersucht und eine Analytkonzentration, beispielsweise die Glucosekonzentration oder die Lactatkonzentration, bestimmt werden. Messergebnisse können mit einer Anzeigeeinrichtung 7 des Geräts 1, beispielsweise einer Flüssigkristallanzeige, angezeigt und in einem nicht dargestellten Speicher des Geräts 1 abgelegt werden.

Der Speicher bildet zusammen mit einer Steuer- und Auswerteeinheit 8, beispielsweise einem Mikroprozessor, und der Anzeigeeinrichtung 7 eine elektronische Baugruppe, welche beispielsweise die Auswertung und Darstellung von Auswertungsergebnissen einer Serie von Messergebnissen ermöglicht, die zusammen mit Datum und Uhrzeit der zugrundeliegenden Messung in dem Speicher gespeichert werden können.
Das Analysehandgerät 1 enthält zusätzlich eine mechanische Baugruppe, die zumindest eine Transporteinrichtung 9 für Testelemente umfasst. Bei dem dargestellten Ausführungsbeispiel bewirkt die Transporteinrichtung 9 ein Aufwickeln des Trägerbands 3 auf eine Aufwickelspule der Kassette 2 und ein Abwickeln von einer Vorratsspule 10, also einen Bandtransport. Bei einem Analysehandgerät, das wie das dargestellte Ausführungsbeispiel zugleich ein Stechgerät ist, enthält die mechanische Baugruppe zusätzlich einen Stechantrieb 11, um Lanzetten 4 in eine Stechbewegung zu versetzen. Der Stechantrieb 11 ist genauso genommen eine weitere Transporteinrichtung, da eine Lanzette bei einem Stich in Stichrichtung bewegt bzw. Transportiert wird. Bevorzugt enthält die mechanische Baugruppe zusätzlich auch einen Elektromotor 12. Prinzipiell kann die von dem Elektromotor an die Transporteinrichtung 9 und den Stechantrieb 11 gelieferte Kraft aber auch von einem Benutzer aufgebracht werden.
Der Stechantrieb 11 enthält vorzugsweise einen mechanischen Energiespeicher, beispielsweise ein Federelement aus Kunststoff oder Metall, der bei Bedarf die für einen schnellen Lanzettenstich benötigte kinetische Energie freisetzt. Ein solcher mechanischer Energiespeicher kann vorteilhaft von dem Elektromotor 12 des Analysehandgeräts 1 aufgeladen werden, beispielsweise indem eine Feder gespannt wird. Der Stechantrieb 11 kann beispielsweise eine Stichbewegung in der in der WO 2008/083844 A1 beschriebenen Weise bewirken.
Das dargestellte Analysehandgerät 1 hat ein erstes Betätigungselement 13, durch dessen Betätigung das Gerät 1 in einen ersten aktiven Zustand versetzt, die mechanische Baugruppe aktiviert und eine Messung vorbereitet wird. Das erste Betätigungselement 13 ist bevorzugt als eine Abdeckung einer Geräteöffnung ausgebildet, an der eine Probenaufnahme erfolgt. Das erste Betätigungselement 13 wird also dadurch betätigt, dass es aus einer die Geräteöffnung abdeckenden Ausgangsposition in eine andere Position bewegt wird, in der die Geräteöffnung freigelegt ist.

Die Vorteile eines integrierten Systems, d. h. eines Handgeräts mit Stech- und Messfunktion, kommen besonders deutlich zur Geltung, wenn die Zeit zwischen Einschalten des Geräts und Messwertanzeige möglichst kurz ist. Andererseits muss die Anzeige des Ergebnisses sicher sein. Vor einem Stich sollte deshalb die Funktionsfähigkeit des Geräts und des darin eingelegten Verbrauchsmaterials überprüft werden. Bevorzugt werden die entsprechenden Tests automatisch durchgeführt, sobald das Gerät in den ersten aktiven Zustand versetzt wird.

Zum Vorbereiten und Durchführen einer Messung könnten beispielsweise einige oder alle der folgenden Schritte durchgeführt werden:
- Freilegen einer Geräteöffnung zum Aufnehmen einer Probe,
- Display Test
- Überprüfen von Codierung und Daten von Verbrauchsmitteln, beispielsweise durch Einlesen von Daten eines Magazins und Prüfung durch Berechnen einer Prüfsumme,
- Prüfen der Messeinrichtung, beispielsweise zum Ermitteln eventueller Defekte oder Verschmutzungen, insbesondere eines optischen Pfads für photometrische Messungen,
- Leermessung eines Testfeldes vor Probenaufnahme
- Messung von Gerätetemperatur und/oder Luftfeuchte,
- Sicherheitsabfrage zum Erkennen eines versehentlichen Aktivierens der Geräts ohne Beabsichtigen einer Messung,
- Aktivieren der mechanischem Baugruppe, insbesondere Bewegen einer Lanzette in ihre Stechposition und Spannen eines Stechantriebs,
- Auslösen eines Stichs,
- Probenaufnahme,
- Durchführen einer Messung,
- Berechnen eines Konzentrationswertes aus einem Messsignal,
- Anzeigen des Messergebnisses,
- Deaktivieren der mechanischen Baugruppe und des Geräts.

Beispielsweise kann das erste Betätigungselement 13 schwenkbar oder verschiebbar an dem Gerätegehäuse 14 befestigt sein. Das erste Betätigungselement 13 kann beispielsweise als Schutzkappe ausgebildet sein und das Innere des Geräts 1 vor Verschmutzung schützen. Indem das erste Betätigungselement 13 als eine Abdeckung der für eine Probenaufnahme vorgesehenen Geräteöffnung ausgeführt ist, wird ein hoher Benutzerkomfort erreicht, da die mechanische Baugruppe nur dann aktiviert wird, wenn ein Benutzer auch tatsächlich eine Messung vornehmen möchte, da der Benutzer in der Regel nur dann die Gehäuseöffnung freilegen wird. Ein wichtiger Vorteil des als Abdeckung ausgebildeten Betätigungselements 13 ist dabei insbesondere auch, dass ein Benutzer die Zeit zwischen dem Bewegen der Abdeckung und dem Anlegen eines Fingers an die Gehäuseöffnung nicht als Wartezeit empfindet, dennoch aber zwischen dem Bewegen der Abdeckung und dem Anlegen eines Fingers an die Gehäuseöffnung in der Regel genug Zeit ist, um das Gerät auf eine Messung vorzubereiten. Ein Benutzer hat deshalb den Eindruck, dass eine Messung "sofort" gestartet werden kann.

Wenn ein Benutzer keine Messung durchführen möchte, sondern beispielsweise lediglich Geräteeinstellungen ändern oder sich vorhandene Messergebnisse anzeigen lassen möchte, kann er das Gerät 1 durch Betätigung eines zweiten Betätigungselements 15, das beispielsweise als eine Taste ausgebildet sein kann, in einen zweiten aktiven Zustand versetzen und die Anzeigeeinrichtung 7 ohne Aktiveren der mechanischen Baugruppe einschalten. Auf der die Anzeigeeinrichtung 7 tragenden Vorderseite des Handgeräts 1 sind bei dem dargestellten Ausführungsbeispiel weitere Betätigungselemente 16 angeordnet. Diese können beispielsweise verwendet werden, um Befehle aus einem von der Anzeigeeinrichtung 7 angezeigten Bedienmenü auszuwählen. Die Anzeigeeinrichtung 7 kann dabei als Sensorfeld ausgebildet sein und ein zusätzliches Betätigungselement bilden. Die zur Probenaufnahme vorgesehene Geräteöffnung bleibt bei dem dargestellten Ausführungsbeispiel also verschlossen, wenn das Gerät durch Betätigen des zweiten Betätigungselements 15 in den zweiten aktiven Zustand versetzt wird.

Wenn sich das Analysehandgerät 1 in dem ersten aktiven Zustand befindet, kann durch Betätigen eines seiner Betätigungselemente eine Messung gestartet werden, bei dem dargestellten Ausführungsbeispiel also ein Lanzettenstich ausgelöst werden. Bevorzugt ist hierfür ein drittes Betätigungselement 17 vorgesehen, das an der Geräteöffnung angeordnet ist. Das dritte Betätigungselement 17 wird durch Anlegen eines Körperteils, in dem eine Stichwunde erzeugt werden soll, an die Geräteöffnung betätigt. Bevorzugt enthält das dritte Betätigungselement 17 einen Temperatur- und/oder Drucksensor, der nicht nur das Anlegen eines Körperteils feststellt, sondern zusätzlich auch überprüft, ob für eine Probenentnahme günstige Bedingungen vorliegen, beispielsweise der Druck, mit dem das Körperteil auf der Geräteöffnung lastet, oder die Temperatur des Körperteils in einem vorgegebenen Bereich liegen. Das Analysehandgerät 1 hat also mehrere Betätigungselemente 13, 15, 16, 17 zum Bedienen des Analysehandgeräts 1.

Bevorzugt deckt das erste Betätigungselement 13 in seiner die Geräteöffnung abdeckenden Ausgangsposition das dritte Betätigungselement 17 ab, so dass dieses im inaktiven Zustand des Geräts 1 geschützt ist.

Nach Abschluss einer Messung geht das Analysehandgerät 1 selbsttätig, also ohne Zutun eines Benutzers, in den zweiten aktiven Zustand über, so dass ein Benutzer zusätzliche Gerätefunktionen nutzen, beispielsweise Auswertungen einer Serie von Messungen betrachten und so seinen Behandlungsfortschritt überprüfen, oder Geräteeinstellungen ändern kann, beispielsweise Weck- oder Erinnerungsfunktionen auf andere Uhrzeiten verstellen kann. Aus dem zweiten aktiven Zustand geht das Gerät in einen inaktiven Zustand über, in dem die Anzeigeeinrichtung abgeschaltet ist. Aus dem zweiten aktiven Zustand kann das Gerät durch Betätigen eines dafür vorgesehenen Betätigungselements in den inaktiven Zustand versetzt, also abgeschaltet werden. Bevorzugt geht das Gerät aus dem zweiten aktiven Zustand auch selbsttätig in den inaktiven Zustand über, wenn während einer vorgegebenen Zeitspanne kein Betätigungselement betätigt wurde. In diesem Fall wird angenommen, dass der Benutzer das Ausschalten des Geräts vergessen hat. Zur Vermeidung unnötigen Energieverbrauchs schaltet sich das Gerät dann selbsttätig ab. Die vorgegebene Zeitspanne kann vom Hersteller des Geräts fest vorgegeben werden und beispielsweise zwischen einer halben und zwei Minuten betragen.

Der Elektromotor 12 und die elektronische Baugruppe mit der Anzeigeeinrichtung 7 und der Steuer- und Auswerteeinheit 8 werden von einer oder mehreren Batterien 18 gespeichert, die in ein dafür vorgesehenes Fach des Geräts 1 eingelegt und bei Bedarf ausgetauscht werden können.

Wenn ein Benutzer nach Aktivieren der mechanischen Baugruppe keine Messung durchführen möchte, beispielsweise weil das erste Betätigungselement 13 versehentlich betätigt wurde, kann die mechanische Baugruppe durch erneutes Betätigen des ersten Betätigungselements 13 deaktiviert werden. Durch erneutes Betätigen des ersten Betätigungselements 13 wird die für eine Probenaufnahme vorgesehene Geräteöffnung wieder verschlossen. Dabei wird die mechanische Baugruppe deaktiviert und das Gerät geht in den zweiten aktiven Zustand über. Bei diesem Deaktivieren der meachnischen Baugruppe wird ein Stich ausgelöst. An der Innenseite des ersten Betätigungselements 13, d.h. der Abdeckung der Geräteöffnung, kann eine Vertiefung sein, so dass die Lanzette die übliche Stichbewegung ausführen kann.

### Bezugszeichen

- 1: Analysehandgerät
- 2: Magazin
- 3: Trägerband
- 4: Lanzetten
- 5: Testelemente
- 6: Sensor
- 7: Anzeigeeinrichtung
- 8: Steuer- und Auswerteeinheit
- 9: Transporteinrichtung
- 10: Vorratsspule
- 11: Stechantrieb
- 12: Elektromotor
- 13: Erstes Betätigungselement
- 14: Gerätegehäuse
- 15: Zweites Betätigungselement
- 16: Weitere Betätigungselemente
- 17: Drittes Betätigungselement
- 18: Batterien
- 20: Gehäusedeckel

## Patentansprüche

1. Analysehandgerät mit
einer mechanischen Baugruppe, die eine Transporteinrichtung (9) für analytische Verbrauchselemente (4, 5) umfasst,
einer Anzeigeeinrichtung (7),
einem Sensor (6) zur Untersuchung einer durch einen Lanzettenstich gewonnenen Körperflüssigkeitsprobe,
einem Speicher zum Speichern einer Serie von Messergebnissen,
einem ersten Betätigungselement (13), durch dessen Betätigung das Gerät (1) in einen ersten aktiven Zustand versetzt werden kann, die mechanische Baugruppe aktiviert und eine Messung vorbereitet werden kann, wobei in dem ersten aktiven Zustand durch Betätigen eines der Betätigungselemente (13, 15, 16, 17) eine Messung gestartet werden kann, und einem zweiten Betätigungselement (15), durch dessen Betätigung das Gerät (1) in einen zweiten aktiven Zustand versetzt werden kann,
**dadurch gekennzeichnet, dass**
durch Betätigen des zweiten Betätigungselements (15) die Anzeigeeinrichtung (7) ohne Aktivieren der mechanischen Baugruppe eingeschaltet werden kann, und durch Betätigen des ersten Betätigungselements (13) die Anzeigeeinrichtung (7) eingeschaltet werden kann.

2. Analysehandgerät nach Anspruch 1, **dadurch gekennzeichnet, dass** das erste Betätigungselement (13) eine Abdeckung einer Geräteöffnung ist, an der eine Probenaufnahme erfolgen kann.

3. Analysehandgerät nach Anspruch 2, **dadurch gekennzeichnet, dass** die Abdeckung schwenkbar ist.

4. Analysehandgerät nach Anspruch 2 oder 3, **dadurch gekennzeichnet, dass** zum Auslösen einer Messung ein drittes Betätigungselement (17) vorgesehen ist, das an der Geräteöffnung angeordnet ist und durch Anlegen eines Körperteils, in dem eine Stichwunde erzeugt werden soll, an die Geräteöffnung betätigt werden kann.

5. Analysehandgerät nach Anspruch 4, **dadurch gekennzeichnet, dass** das erste Betätigungselement (13) in seiner die Geräteöffnung abdeckenden Ausgangsposition das dritte Betätigungselement (17) abdeckt.

6. Analysehandgerät nach einem der vorstehenden Ansprüche, **dadurch gekennzeichnet, dass** die mechanische Baugruppe einen Stechantrieb (11) umfasst, um Lanzetten (4) in eine Stechbewegung zu versetzen.

7. Analysehandgerät nach Anspruch 6, **dadurch gekennzeichnet, dass** eine Messung vorbereitet werden kann, indem der Stechantrieb (11) an eine Lanzette (4) ankoppelt.

8. Analysehandgerät nach einem der vorstehenden Ansprüche, **dadurch gekennzeichnet, dass** die mechanische Baugruppe einen Elektromotor (12) umfasst.

9. Analysehandgerät nach einem der vorstehenden Ansprüche, **dadurch gekennzeichnet, dass** eine Messung verbereitet werden kann, indem ein Verbrauchselement (4, 5) von der Transporteinrichtung (9) aus einem Verbrauchselementevorrat entnommen wird.

10. Analysehandgerät nach einem der vorstehenden Ansprüche, **dadurch gekennzeichnet, dass** in dem zweiten aktiven Zustand Geräteeinstellungen geändert werden können.

11. Analysehandgerät nach einem der vorstehenden Ansprüche, **dadurch gekennzeichnet, dass** in dem zweiten aktiven Zustand Messergebnisse von der Anzeigeeinrichtung (7) angezeigt werden können.

12. Analysehandgerät nach einem der vorstehenden Ansprüche, **dadurch gekennzeichnet, dass** es aus dem zweiten aktiven Zustand selbsttätig in einen inaktiven Zustand übergehen kann, in dem die Anzeigeeinrichtung (7) abgeschaltet ist, wenn während einer vorgegebenen Zeitspanne kein Betätigungselement (13, 15, 16, 17) betätigt wurde.

13. Analysehandgerät nach einem der vorstehenden Ansprüche, **dadurch gekennzeichnet, dass** die mechanische Baugruppe durch Betätigen des ersten Betätigungselements (13) deaktiviert werden kann.

14. System mit einem Analysehandgerät (1) nach einem der vorstehenden Ansprüche und einem in das Gerät einsetzbaren Magazin (2), das einen Verbrauchselementevorrat enthält.

## Claims

1. Handheld analysis device, comprising
a mechanical assembly, which comprises a transport unit (9) for consumable analytical elements (4, 5),
a display unit (7),
a sensor (6) for analyzing a body fluid sample obtained by a lancet prick,
a memory for storing a series of measurement results,
a first actuating element (13), the actuation of which can transfer the device (1) into a first active state, activate the mechanical assembly and can prepare a measurement, wherein in the first active state a measurement can be started by actuating one of the actuating elements (13, 15, 16, 17), and
a second actuating element (15), the actuation of which can transfer the device (1) into a second active state,
**characterized in that**
the display unit (7) can be switched on by actuating the second actuating element (15), without activating the mechanical assembly, and
the display unit (7) can be switched on by actuating the first actuating element (13).

2. Handheld analysis device according to claim 1, **characterized in that** the first actuating element (13) is a cover for a device opening on which a sample can be taken up.

3. Handheld analysis device according to claim 2, **characterized in that** the cover can be pivoted.

4. Handheld analysis device according to claim 2 or 3, **characterized in that** a third actuating element (17) is provided for triggering a measurement, the third actuating element being disposed on the device opening and can be actuated by placing a body part on which a puncture wound is to be generated against the device opening.

5. Handheld analysis device according to claim 4, **characterized in that** in the starting position, in which it covers the device opening, the first actuating element (13) covers the third actuating element (17).

6. Handheld analysis device according to any one of the preceding claims,
**characterized in that** the mechanical assembly comprises a lancing drive (11) so as to cause the lancets (4) to perform a piercing movement.

7. Handheld analysis device according to claim 6, **characterized in that** a measurement is prepared by coupling the lancing drive (11) to a lancet (4).

8. Handheld analysis device according to any one of the preceding claims,
**characterized in that** the mechanical assembly comprises an electric motor (12).

9. Handheld analysis device according to any one of the preceding claims,
**characterized in that** a measurement can be prepared **in that** a consumable element (4, 5) is removed by the transport unit (9) from a consumable element supply.

10. Handheld analysis device according to any one of the preceding claims,
**characterized in that** device settings can be modified in the second active state.

11. Handheld analysis device according to any one of the preceding claims,
**characterized in that** measurement results can be displayed by the display unit (7) in the second active state.

12. Handheld analysis device according to any one of the preceding claims,
**characterized in that** it can automatically transition from the second active state into an inactive state, in which the display unit (7) is switched off when no actuating element (13, 15, 16, 17) was actuated during a predefined time period.

13. Handheld analysis device according to any one of the preceding claims,
**characterized in that** the mechanical assembly (7) can be deactivated by actuating the first actuating element (13).

14. System comprising a handheld analysis device (1) according to any one of the preceding claims and a magazine (2) that can be inserted in the device, the magazine containing a consumable element supply.

## Revendications

1. Appareil d'analyse portatif comprenant un module mécanique pourvu d'un dispositif de transport (9) pour des éléments consommables d'analyse (4, 5),
un dispositif d'affichage (7),
un capteur (6) pour analyser un échantillon de liquide biologique obtenu par une piqûre de lancette,
une mémoire pour stocker une série de résultats de mesure,
un premier élément de commande (13), dont l'actionnement permet de mettre l'appareil (1) dans un premier état actif, afin d'activer le module mécanique et de préparer une mesure, une mesure étant lancée dans le premier état actif par l'actionnement d'un des éléments de commande (13, 15, 16, 17), et
un second élément de commande (15) dont l'actionnement permet de mettre l'appareil (1) dans un second état actif,
**caractérisé en ce que**,
l'actionnement du second élément de commande (15) permet de mettre en marche le dispositif d'affichage (7) sans activer le module mécanique, et l'actionnement du premier élément de commande (13) permet de mettre en marche le dispositif d'affichage (7).

2. Appareil d'analyse portatif selon la revendication 1, **caractérisé en ce que** le premier élément d'actionnement (13) est un chapeau d'une ouverture de l'appareil, au niveau de laquelle une réception d'échantillon peut s'effectuer.

3. Appareil d'analyse portatif selon la revendication 2, **caractérisé en ce que** le chapeau peut pivoter.

4. Appareil d'analyse portatif selon la revendication 2 ou 3, **caractérisé en ce que**, pour déclencher une mesure, un troisième élément de commande (17) est prévu, lequel est disposé au niveau de l'ouverture de l'appareil et peut être actionné par un appui d'une partie du corps, dans laquelle une perforation doit être effectuée, au niveau de l'ouverture de l'appareil.

5. Appareil d'analyse portatif selon la revendication 4, **caractérisé en ce que** le premier élément de commande (13) recouvre le troisième élément de commande (17) dans sa position initiale recouvrant l'ouverture de l'appareil.

6. Appareil d'analyse portatif selon l'une quelconque des revendications précédentes, **caractérisé en ce que** le module mécanique comprend un dispositif d'actionnement de piqueur (11) destiné à déplacer des lancettes (4) dans un mouvement de perforation.

7. Appareil d'analyse portatif selon la revendication 6, **caractérisé en ce qu'**une mesure peut être préparée en couplant le dispositif d'actionnement de piqueur (11) à une lancette (4).

8. Appareil d'analyse portatif selon l'une quelconque des revendications précédentes, **caractérisé en ce que** le module mécanique comprend un moteur électrique (12).

9. Appareil d'analyse portatif selon l'une quelconque des revendications précédentes, **caractérisé en ce qu'**une mesure peut être préparée en prélevant par le dispositif de transport (9) un élément consommable (4,5) à partir d'une réserve d'éléments consommables.

10. Appareil d'analyse portatif selon l'une quelconque des revendications précédentes, **caractérisé en ce que** des réglages de l'appareil peuvent être modifiés dans le second état actif.

11. Appareil d'analyse portatif selon l'une quelconque des revendications précédentes, **caractérisé en ce que** des résultats de mesure peuvent être affichés par le dispositif d'affichage (7) dans le second état actif.

12. Appareil d'analyse portatif selon l'une quelconque des revendications précédentes, **caractérisé en ce qu'**il peut passer automatiquement du second état actif dans un état inactif, dans lequel le dispositif d'affichage (7) est mis hors circuit, lorsque, pendant un intervalle de temps prescrit, aucun élément de commande (13, 15, 16, 17) n'a été actionné.

13. Appareil d'analyse portatif selon l'une quelconque des revendications précédentes, **caractérisé en ce que** le module mécanique peut être désactivé par un actionnement du premier élément de commande (13).

14. Système comprenant un appareil d'analyse portatif (1) selon l'une quelconque des revendications précédentes et un magasin (2) pouvant être inséré dans l'appareil, lequel contient une réserve d'éléments consommables.
